# EUROPÄISCHE PATENTSCHRIFT

(11) **EP 2 059 279 B1**
(45) Veröffentlichungstag und Bekanntmachung des Hinweises auf die Patenterteilung: **17.02.2010**
(21) Anmeldenummer: 07801928.8
(22) Anmeldetag: 27.08.2007
(51) Int. Cl.: A61M 1/36

(54) **BLUTBEHANDLUNGSGERÄT UND VERFAHREN ZUM ENTLEEREN EINES BLUTSCHLAUCHSATZES EINES BLUTBEHANDLUNGSGERÄTES**
BLOOD PROCESSING DEVICE AND METHOD FOR PURGING A SET OF BLOOD LINES ON A BLOOD PROCESSING DEVICE
APPAREIL DE TRAITEMENT SANGUIN ET PROCÉDÉ POUR PURGER UN JEU DE LIGNES À SANG D'UN APPAREIL DE TRAITEMENT SANGUIN

(30) Priorität: 07.09.2006 DE 102006042120
(43) Veröffentlichungstag der Anmeldung: 20.05.2009
(73) Patentinhaber: Fresenius Medical Care Deutschland GmbH, 61352 Bad Homburg (DE)
(72) Erfinder: GRONAU, Sören, 64569 Nauheim (DE); HÄCKER, Jürgen, 61267 Neu-Anspach (DE); GÜNTHER, Götz, 61440 Oberursel (DE); FISCHER, Max, 60323 Frankfurt am Main (DE); NOACK, Joachim, 97616 Bad Neustadt (DE)
(74) Vertreter: Herrmann, Uwe
(86) Internationale Anmeldenummer: PCT/EP2007/007505
(87) Internationale Veröffentlichungsnummer: WO 2008/028579

(56) Entgegenhaltungen:
- EP-A- 0 578 175
- EP-B- 0 830 153
- EP-B- 1 161 271
- DE-A1- 10 245 619

## Beschreibung

Die Erfindung betrifft ein Verfahren zum Entleeren eines Blutschlauchsatzes eines Blutbehandlungsgerätes zur extrakorporalen Blutbehandlung mit einem Membranfilter, das eine erste Kammer, nämlich eine Blutkammer, und eine von dieser durch eine Membran getrennte zweite Kammer aufweist, wobei die erste Kammer im Betrieb des Blutbehandlungsgerätes von Blut durchströmt wird und wobei die zweite Kammer im Betrieb des Blutbehandlungsgerätes von Filtrat durchströmt wird, und wobei der Blutschlauchsatz einen arteriellen und einen venösen Blutschlauch umfasst, die beide mit der ersten Kammer des Membranfilters in Verbindung stehen und die im Betrieb des Blutbehandlungsgerätes Blut vom Patienten zum Membranfilter und vom Membranfilter zum Patienten führen, sowie mit einer Substituatleitung, die in den arteriellen und/oder in den venösen Blutschlauch mündet, und mit einer Substituatpumpe, die mit der Substituatleitung derart in Verbindung steht, dass sie Substituat aus einer Substituatquelle durch die Substituatleitung fördert. Bei dem genannten Membranfilter kann es sich beispielsweise um einen Hämodiafilter oder um einen Hämofilter handeln.

Aus dem Stand der Technik sind verschiedene Verfahren zum Entleeren eines Blutschlauchsatzes bekannt. Aus der EP 1 161 271 A1 ist es bekannt, zum Zwecke der Entleerung des Blutschlauchsatzes auf der Dialysatseite befindliche Dialysatpumpen derart zu betreiben, dass sich über die Membran des Dialysators ein Druckgefälle einstellt, mittels dessen die in dem Blutschlauchsatz befindliche Flüssigkeit über die Membran des Dialysators auf die Dialysatseite, das heißt in die zweite Kammer transportiert und von dort aus mittels abführender Leitungen abgezogen wird. Mit dem Blutschlauchsatz steht ein Substituat-Beutel in Verbindung, der aufgrund der Entleerung des Blutschlauchsatzes bzw. aufgrund des darin befindlichen geringen Druckes kollabiert. Sobald der Druck in dem Blutschlauchsatz einen bestimmten Grenzwert unterschreitet, wird ein Ventil geöffnet, über das Luft in den Blutschlauchsatz gesogen wird, bis der in dem Blutschlauchsatz befindliche Druck dem Atmosphärendruck entspricht.

Während der Entleerung des Blutschlauchsatzes über die Membran des Dialysators ist die Blutpumpe in Betrieb, bis die Flüssigkeit aus dem Blutschlauchsatz entfernt wurde.

Aus der EP 0 830 153 A1 ist es bekannt, mittels Luft Flüssigkeit aus dem Blutschlauchsatz sowie auch aus dem Dialysatkreislauf zu verdrängen, bevor die entsprechenden Schlauchleitungen von dem Dialysator getrennt werden.

Die DE 34 42 744 A1 offenbart schließlich ein Verfahren, bei dem mit Hilfe einer Ringleitung im Blutschlauchsatz und einer Luftquelle die im Blutschlauchsatz befindliche Flüssigkeit verdrängt wird. Gegenstand der Patentanmeldung ist es, eine Leckprüfung bzw. einen Druckfest am Dialysator durchführen zu können.

Der vorliegenden Erfindung liegt die Aufgabe zugrunde, das nach Abschluß der Blutrückgabe in den Patienten bzw. das nach Abschluß der Blutbehandlung in dem Blutschlauchsatz verbleibende Gemisch aus Blut und Verdrängungsmittel bzw. Substituat auf einfache und effiziente Weise aus dem Blutschlauchsatz zu entfernen.

Diese Aufgabe wird durch ein Verfahren mit den Merkmalen des Anspruchs 1 gelöst. Danach ist vorgesehen, dass zum Zwecke des Entleerens des Blutschlauchsatzes der arterielle und der venöse Blutschlauch derart miteinander verbunden werden, dass ein die Blutkammer, den arteriellen und den venösen Blutschlauch umfassender Kreislauf entsteht, dass die Substituatleitung von der Substituatquelle dekonnektiert wird und dass mittels der Subsitutatpumpe Luft in den Blutschlauchsatz gepumpt wird, so dass in dem Blutschlauchsatz ein Überdruck entsteht, durch den Flüssigkeit von der Blutkammer über die Membran in die zweite Kammer des Membranfilters verdrängt wird.

Gegenstand der Erfindung ist es somit, dass die ohnehin vorhandene Substituatpumpe dazu genutzt wird, zum Zwecke der Entleerung des Blutschlauches Luft in den Blutschlauchsatz zu fördern, woraufhin das Gemisch aus Blut und Substituat bzw. Verdrängungsmittel über die Membran von der Blutkammer in die zweite Kammer des Membranfilters verdrängt wird und von dieser über abführende Leitungen abgeleitet wird. Auf diese Weise ist es möglich, mit vergleichsweise geringem Aufwand eine effiziente Verdrängung der Flüssigkeit aus dem Blutschlauchsatz zu bewirken.

Das erfindungsgemäße Verfahren findet ohne Beteiligung des Patienten, d. h. nach Abschluss der Patientenbehandlung bei dekonnektiertem Patienten statt.

Sobald das erfindungsgemäße Entleerungsverfahren beendet ist, kann der Blutschlauchsatz, bei dem es sich üblicherweise um einen Einmalartikel (Disposable) handelt, verworfen werden. Durch die Entleerung des Blutschlauchsatzes können Entsorgungskosten aufgrund der geringeren Abfallmasse reduziert werden. Des weiteren sprechen hygienische Gründe für eine vollständige Entleerung des Blutschlauchsatzes vor dessen Entsorgung.

Zur Durchführung des erfindungsgemäßen Verfahrens weist das Blutbehandlungsgerät eine Steuerung auf. Es kann sich dabei um ein zu dem ohnehin vorhandenen Steuergerät des Blutbehandlungsgerätes zusätzliches Steuergerät handeln. Denkbar ist auch, dass die genannte Steuerung in dem ohnehin vorhandenen Steuergerät zum Betrieb des Blutbehandlungsgerätes verwirklicht ist, wobei dieses Steuergerät in diesem Fall vorzugsweise dahingehend erweitert ist, dass es die Ansteuerung der Substituatpumpe und/oder des Drucksensors und/oder möglicher Ventile im arteriellen oder venösen Blutschlauch bzw. Zweig vornimmt und/oder automatisch der Lufteintritt in die Substituatleitung des Blutschlauchsatzes ermöglicht wird. Diese Erweiterungen können beispielsweise durch eine Softwareänderung realisiert werden.

In diesem Zusammenhang ist darauf hinzuweisen, dass die Dekonnektion der Substituatleitung von der Substituatquelle vorzugsweise manuell durch das Behandlungspersonal erfolgt. Denkbar ist jedoch auch, eine Öffnung gegenüber der Umgebungsluft zu automatisieren und über die Steuerung auszuführen. Unter dem Begriff "Dekonnektion" ist im Rahmen der vorliegenden Erfindung nicht nur die körperliche Trennung, d. h. das Abkoppeln der Substituatleitung von der Substituatquelle, sondern z. B. auch das Verschließen der Leitung zwischen Substituatpumpe und Substituatquelle zu verstehen.

In weiterer Ausgestaltung der Erfindung ist vorgesehen, dass die Substituatpumpe mit Umgebungsluft beaufschlagt wird, die sodann durch die Substitutatpumpe in den Blutschlauchsatz gefördert wird.

Die Substituatleitung kann als Prädilutionsleitung und/oder als Postdilutionsleitung ausgeführt sein. Derartige Leitungen werden genutzt, um während des Betriebes des Blutbehandlungsgerätes das Blut vor dem Eintritt in die Blutkammer (Prädilution) oder nach dem Austritt aus der Blutkammer (Postdilution) zu verdünnen. Auch eine kombinierte Verfahrensweise von Prä- und Postdilution ist denkbar.

Die Substituatleitung kann sich bei diesem Ausführungsbeispiel der Erfindung in eine Prädilutionsleitung und in eine Postdilutionsleitung verzweigen, die einerseits vor dem Membranfilter in den arteriellen Blutschlauch mündet und die andererseits nach dem Membranfilter in den venösen Blutschlauch mündet.

Vorzugsweise ist vorgesehen, dass bei der Entleerung des Blutschlauchsatzes eine der beiden Leitungen (Prädilutionsleitung, Postdilutionsleitung) abgesperrt wird, so dass die in der Substituatpumpe komprimierte Luft nur durch die andere dieser Leitungen in den Blutschlauchsatz gefördert wird.

Denkbar ist beispielsweise, dass sowohl die Prädilutionsleitung als auch die Postdilutionsleitung mit Absperrventilen versehen sind, mittels derer die Leitungen verschließbar sind. Denkbar ist beispielsweise, dass das Ventil der Prädilutionsleitung verschlossen wird und die durch die Substituatpumpe komprimierte Luft somit nur über die Postdilutionsleitung in den Blutschlauchsatz gefördert wird.

In weiterer Ausgestaltung der Erfindung ist vorgesehen, dass eine Blutpumpe derart angeordnet ist, dass sie mit einem der Blutschläuche, vorzugsweise mit dem arteriellen Blutschlauch, derart in Verbindung steht, dass im Betrieb des Blutbehandlungsgerätes Blut durch den Blutschlauchsatz gefördert wird, wobei die Blutpumpe während des Entleerens des Blutschlauchsatzes wenigstens zeitweise in Betrieb ist. Die Blutpumpe kann somit bei der Entleerung des Blutschlauchsatzes unterstützend eingreifen.

In weiterer Ausgestaltung der Erfindung ist vorgesehen, dass die mittels der Substituatpumpe geförderte Luft über die Prädilutionsleitung oder Postdilutionsleitung in den arteriellen oder venösen Blutschlauch gefördert wird und in diesem derart aufgeteilt wird, dass die Luft bzw. die von der Luft verdrängte Flüssigkeit sowohl über den arteriellen als auch über den venösen Blutschlauch in die Blutkammer eintritt. Möglich ist es beispielsweise, dass die Luft über die Postdilutionsleitung in den venösen Blutschlauch eingeführt wird und sich dort aufteilt. Ein Teil der Luft gelangt entgegen der im Betrieb des Blutbehandlungsgerätes üblichen Strömungsrichtung von einer Seite in die Blutkammer und der andere Teil der Luft gelangt über den Kurzschluß zwischen arteriellem und venösem Blutschlauch durch die arterielle Leitung von der anderen Seite in die Blutkammer. Auf diese Weise ist eine vollständige Entleerung des Blutschlauchsatzes mit vergleichsweise einfachen Mitteln möglich.

Gemäß einer weiteren Ausgestaltung der Erfindung kann vorgesehen sein, dass die Substituatpumpe nach einer vorbestimmten Zeitspanne ab dem Einschaltzeitpunkt wieder abgeschaltet wird. Es ist somit möglich, dass nach einer definierten Zeitspanne die Substituatpumpe stoppt und der Blutschlauchsatz als leer betrachtet et wird.

Ebenso ist es denkbar, dass ein Drucksensor vorgesehen ist, der derart angeordnet ist, dass er den Druck in dem arteriellen und/oder in dem venösen Blutschlauch misst und dass eine Steuereinrichtung vorgesehen ist, an die der Drucksensor angeschlossen ist und die die Substituatpumpe abschaltet, sobald der mittels des Drucksensors erfaßte Druck einen Grenzwert übersteigt. Eine nasse Membran ist für Flüssigkeit wesentlich permeabler als für Luft und somit kann zum Zeitpunkt der vollständigen Entleerung ein signifikanter Druckanstieg gemessen werden, der in diesem Ausführungsbeispiel der Erfindung dazu genutzt wird, die Substituatpumpe abzuschalten, weil der Blutschlauchsatz als leer identifiziert wird.

In einer weiteren Ausgestaltung der Erfindung ist es möglich, dass von dem arteriellen und/oder von dem venösen Blutschlauch ein Leitungsabschnitt abzweigt und dass dieser Leitungsabschnitt mit dem arteriellen oder venösen Blutschlauch oder einem daran angeordneten weiteren Leitungsabschnitt verbunden wird, um den Kreislauf herzustellen. Dabei ist des weiteren vorgesehen, dass der arterielle und/oder venöse Blutschlauch stromabwärts der Einmündung des abzweigenden Leitungsabschnitts abgesperrt wird, um zu verhindern, dass Flüssigkeit aus dem arteriellen oder venösen Blutschlauch austritt.

Die Erfindung betrifft ferner ein Blutbehandlungsgerät mit einem extrakorporalen Blutkreislauf, der geeignet ist zur Aufnahme eines Blutschlauchsatzes sowie eines Membranfilters mit einer ersten Kammer und einer von dieser durch eine Membran getrennten zweiten Kammer, wobei die erste Kammer in Betrieb des Blutbehandlungsgerätes von Blut und die zweite Kammer in Betrieb des Blutbehandlungsgerätes von Filtrat durchströmt wird. Das Blutbehandlungsgerät ist **dadurch gekennzeichnet, dass** es eine Substituatpumpe sowie eine Steuereinheit aufweist, die die Substituatpumpe zum Entleeren des Blutschlauchsatzes derart ansteuert, dass mittels der Substituatpumpe Luft oder Gas in den Blutschlauchsatz gepumpt wird, so dass in dem Blutschlauchsatz ein Überdruck entsteht, durch den Flüssigkeit über die Membran des Membranfilters von der ersten in die zweite Kammer des Membranfilters verdrängt wird.

In bevorzugter Ausgestaltung der Erfindung ist vorgesehen, dass die Substituatpumpe derart ausgeführt ist, dass sie nach der Dekonnektion der Substituatleitung Umgebungsluft ansaugt und in den Blutschlauchsatz fördert.

In weiterer Ausgestaltung der Erfindung ist eine Blutpumpe vorgesehen, die seitens der Steuereinheit zum Zwecke der Entleerung des Blutschlauchsatzes derart angesteuert wird, dass sie während des Entleerens des Blutschlauchsatzes permanent oder zeitweise in Betrieb ist.

In einer weiteren Ausgestaltung der Erfindung ist die Steuereinheit derart ausgeführt, dass sie die Substituatpumpe nach einer vorbestimmten Zeitspanne ab dem Einschaltzeitpunkt wieder abschaltet. Damit besteht die Möglichkeit, dass die Steuereinheit die Substituatpumpe nach Ablauf einer definierten Zeitspanne anhält und der Blutschlauchsatz sodann als leer betrachtet wird.

In weiterer Ausgestaltung der Erfindung ist ein Drucksensor vorgesehen, der an die Steuereinheit angeschlossen ist und der derart angeordnet ist, dass er den Druck in dem arteriellen und/oder in dem venösen Blutschlauch mißt. In diesem Fall ist vorgesehen, dass die Substituatpumpe von der Steuereinrichtung abgeschaltet wird, sobald der mittels des Drucksensors erfaßte Druck einen Grenzwert übersteigt.

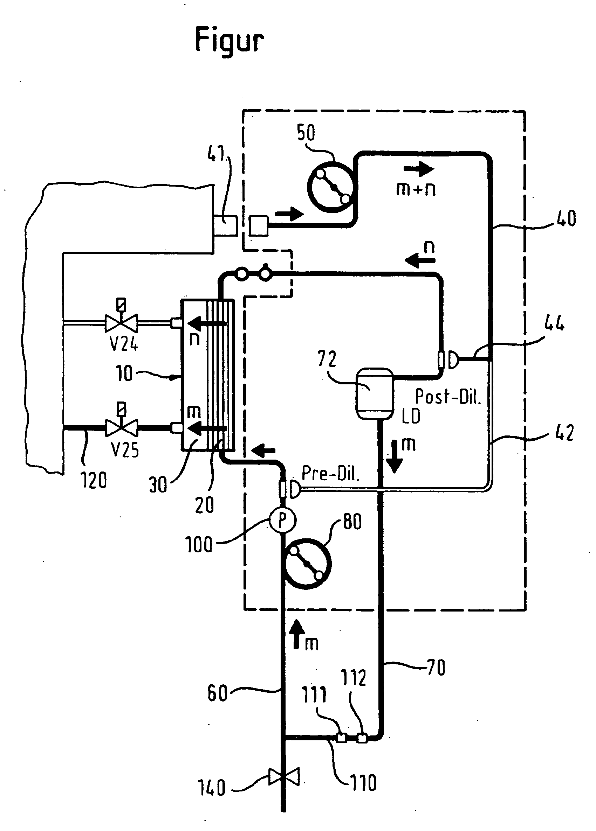

Weitere Einzelheiten und Vorteile der Erfindung werden anhand eines in der Zeichnung dargestellten Ausführungsbeispiels näher erläutert. Die einzige Figur zeigt eine schematische Darstellung eines Blutbehandlungsverfahrens am Beispiel eines Hämodiafiltrationsverfahrens.

Das Blutbehandlungsgerät weist eine Filtratseite und einen extrakorporalen Blutkreislauf auf. Der extrakorporale Blutskreislauf umfasst einen Blutschlauchsatz sowie die erste Kammer 20 des Membranfilters 10, die von der zweiten Kammer 30 durch eine semipermeable Membran getrennt ist. Im Betrieb des Gerätes wird über den arteriellen Blutschlauch 60 Blut von dem Patienten abgezogen, in die erste Kammer 20 eingeführt und über den venösen Blutschlauch 70 sowie die daran befindliche Tropfkammer 72 wieder zurück zum Patienten geführt. Die Förderung des Blutes im extrakorporalen Kreislauf erfolgt über die Blutpumpe 80, die in dem arteriellen Blutschlauch 60 angeordnet ist. Die gestrichelte Linie in der Figur kennzeichnet den als Disposable ausgeführten Teil der Anordnung.

Wie dies weiter aus der Figur hervorgeht, ist eine Substituatpumpe 50 vorgesehen, die sich in der Substituatleitung 40 befindet. Die Substituatleitung weist einen Anschluss auf, über den die Substituatleitung 40 mit einer Substituatquelle verbunden ist. Das Substituat wird in diesem Ausführungsbeispiel in dem Blutbehandlungsgerät online aufbereitet und am Substituatport 41 bereitgestellt. Grundsätzlich ist ebenfalls denkbar, eine von der Blutbehandlungsmaschine unabhängige Substituatquelle vorzusehen.

Im Betrieb fördert die Substituatpumpe 50 Substituat durch die Substituatleitung 40. Die Substituatleitung 40 mündet über eine Prädilutionsleitung 42 und eine Postdilutionsleitung 44 in den Blutschlauchsatz. Im einzelnen ist vorgesehen, dass die Prädilutionsleitung 42 in den arteriellen Blutschlauch 60 und die Postdilutionsleitung 44 in den venösen Blutschlauch 70 mündet. Wie dies aus der Figur ersichtlich ist, mündet die Prädilutionsleitung 42 in einen Leitungsabschnitt zwischen der Blutpumpe 80 und der ersten Kammer des Membranfilters 10. Die Postdilutionsleitung 44 mündet in einen Leitungsabschnitt zwischen dem Membranfilter 10 und der venösen Tropfkammer 72.

Zum Zwecke der Entleerung des die Schläuche 60 und 70 umfassenden Blutschlauchsets wird nun die Substituatleitung 40 vorzugsweise manuell durch das Behandlungspersonal von dem Anschlußport 41 der Substiuatquelle getrennt. Ferner wird der von dem arteriellen Blutschlauch 60 abzweigende Leitungsabschnitt 110 über Konnektoren 111, 112 mit dem venösen Blutschlauch 70 ebenfalls vorzugsweise manuell durch das Behandlungspersonal kurzgeschlossen, so dass ein Kreislauf entsteht, der den arteriellen Blutschlauch 60, den venösen Blutschlauch 70, die venöse Tropfkammer 72 und die erste Kammer 20 des Membranfilters 10 umfaßt. Der nicht zu diesem Kreislauf gehörende Abschnitt des arteriellen Blutschlauches 60, der sich unterhalb der Mündungsstelle der abzweigenden Leitung 110 befindet, wird durch ein Absperrelement 140 verschlossen.

Die Substituatpumpe 50 fördert nun entsprechend der Dekonnektion der Substituatleitung von der Substituatquelle Luft in die Substituatleitung 40 wie dies durch den Pfeil und die Kennzeichnung "m+n" gekennzeichnet ist. Während dessen ist die Prädilutionsleitung 42 über ein geeignetes Ventil verschlossen. Somit wird der gesamte durch die Substituatpumpe 50 geförderte Luftstrom über die Postdilutionsleitung 44 in den venösen Blutschlauch 70 gefördert. Er teilt sich hier in die Teilströme "n" und "m" auf, wobei der Teilstrom "n" bzw. die durch diesen verdrängte Flüssigkeit entgegen der während des Betriebes des Blutbehandlungsgerätes üblichen Strömungsrichtung gemäß der Figur von oben über den Ablaufstutzen in die erste Kammer 20 des Membranfilters 10 eintritt. Der andere Teil der zugeführten Luft, der in der Figur mit dem Zeichen "m" gekennzeichnet ist bzw. die durch diesen verdrängte Flüssigkeit, wird über die venöse Tropfkammer 72 geführt und gelangt über die Konnektoren 111, 112 und den Leitungsabschnitt 110 in den arteriellen Blutschlauch 60 und über diesen über den Zulaufport der ersten Kammer in die erste Kammer 20 des Membranfilters 10.

Die erste Kammer 20 des Membranfilters 10 wird somit beidseitig mit Luft/Flüssigkeit und somit mit Druck beaufschlagt, so dass die in dem Blutschlauchsatz befindliche Flüssigkeit bzw. das Flüssigkeits-/Luftgemisch von der ersten Kammer 20 über die semipermeable Membran in die zweite Kammer 30 des Membranfilters 10 transportiert und sodann mittels der Leitung 120 abgeführt wird.

Während dieses Entleerungsvorgangs kann die in dem arteriellen Blutschlauch 60 befindliche Blutpumpe 80 zur Unterstützung des Entleerungsvorgangs permanent oder zumindest zeitweise laufen. Stromabwärts der Blutpumpe 80 befindet sich in dem arteriellen Blutschlauch 60 der Drucksensor 100, der den Druck in dem arteriellen Blutschlauch 60 während des Entleerungsvorgangs misst. Unterschreitet der gemessene Druckwert einen Grenzwert, wird festgestellt, dass der Blutschlauchsatz entleert ist und die Pumpen 50, 80 werden abgeschaltet.

Alternativ dazu besteht die Möglichkeit, den Entleerungsvorgang nach Ablauf einer bestimmten Zeitspanne ab Beginn des Vorgangs zu beenden.

Das erfindungsgemäße Verfahren bietet eine einfache und effiziente Möglichkeit zur Entleerung eines Blutschlauchsatzes, durch dass das Abfallgewicht verringert wird und die Hygiene verbessert wird. Das erfindungsgemäße Verfahren läuft vorzugsweise automatisch ab, das heißt ein Eingriff durch den Anwender ist abgesehen von der Dekonnektion der Substituatleitung vom Substituat-Anschlussport und dem Kurzschließen der Konnektoren 111, 112 (die vorzugsweise manuell erfolgen) vorzugsweise nicht erforderlich.

## Patentansprüche

1. Verfahren zum Entleeren eines Blutschlauchsatzes eines Blutbehandlungsgerätes mit extrakorporalem Blutkreislauf mit einem Membranfilter (10), der eine erste Kammer (20) und eine von dieser durch eine Membran getrennte zweite Kammer (30) aufweist, wobei die erste Kammer (20) im Betrieb des Blutbehandlungsgerätes von Blut durchströmt wird und wobei die zweite Kammer (30) im Betrieb des Blutbehandlungsgerätes von Filtrat durchströmt wird, und wobei der Blutschlauchsatz einen arteriellen (60) und einen venösen Blutschlauch (70) umfasst, die beide mit der ersten Kammer (20) des Membranfilters (10) in Verbindung stehen und die im Betrieb des Blutbehandlungsgerätes Blut vom Patienten zu dem Membranfilter (10) und vom Membranfilter (10) zum Patienten führen, sowie mit einer Substituatleitung (40), die in den arteriellen (60) und/oder in den venösen Blutschlauch (70) mündet, und einer Substituatpumpe (50), die mit der Substituatleitung (40) derart in Verbindung steht, dass sie Substituat aus einer Substituatquelle durch die Substituatleitung (40) fördert **dadurch gekennzeichnet, dass** zum Zwecke des Entleerens des Blutschlauchsatzes der arterielle (60) und der venöse Blutschlauch (70) derart miteinander verbunden werden, dass ein den Membranfilter (10), den arteriellen (60) und den venösen Blutschlauch (70) umfassender Kreislauf entsteht, dass die Substituatleitung (40) von der Substituatquelle dekonnektiert wird und dass mittels der Subsitutatpumpe (50) Luft oder Gas in den Blutschlauchsatz gepumpt wird, so dass in dem Blutschlauchsatz ein Überdruck entsteht, durch den Flüssigkeit über die Membran des Membranfilters (10) in die zweite Kammer (30) des Membranfilters (10) verdrängt wird.

2. Verfahren nach Anspruch 1, **dadurch gekennzeichnet, dass** die Substituatpumpe (50) mit Umgebungsluft beaufschlagt wird, die durch die Substitutatpumpe (50) in den Blutschlauchsatz gefördert wird.

3. Verfahren nach Anspruch 1 oder 2, **dadurch gekennzeichnet, dass** die Substituatleitung (40) als Prädilutionsleitung (42) und/oder als Postdilutionsleitung (44) ausgeführt ist.

4. Verfahren nach einem der vorhergehenden Ansprüche, **dadurch gekennzeichnet, dass** die Substituatleitung (40) in eine Prädilutionsleitung (42) und in eine Postdilutionsleitung (44) verzweigt und dass bei der Entleerung des Blutschlauchsatzes die Prädilutionsleitung (42) oder die Postdilutionsleitung (44) abgesperrt wird, so dass die in der Substituatpumpe (50) komprimierte Luft nur durch die Prädilutionsleitung (42) oder die Postdilutionsleitung (44) in den Blutschlauchsatz gefördert wird.

5. Verfahren nach einem der vorhergehenden Ansprüche, **dadurch gekennzeichnet, dass** eine Blutpumpe (80) angeordnet ist, die mit einem der Blutschläuche, vorzugsweise mit dem arteriellen Blutschlauch (60), derart in Verbindung steht, dass im Betrieb des Blutbehandlungsgerätes Blut durch den Blutschlauchsatz gefördert wird, und dass die Blutpumpe (80) während des Entleerens des Blutschlauchsatzes permanent oder zeitweise in Betrieb ist.

6. Verfahren nach einem der vorhergehenden Ansprüche, **dadurch gekennzeichnet, dass** die mittels der Substituatpumpe (50) geförderte Luft über die Prädilutionsleitung (42) oder Postdilutionsleitung (44) in den arteriellen (60) oder venösen Blutschlauch (70) gefördert wird und in diesem derart aufgeteilt wird, dass die Luft bzw. die von der Luft verdrängte Flüssigkeit sowohl über den arteriellen (60) als auch über den venösen Blutschlauch (70) in die erste Kammer (20) des Membranfilters (10) eintritt.

7. Verfahren nach einem der vorhergehenden Ansprüche, **dadurch gekennzeichnet, dass** die Substituatpumpe (50) nach einer vorbestimmten Zeitspanne ab dem Einschaltzeitpunkt wieder abgeschaltet wird.

8. Verfahren nach einem der Ansprüche 1 bis 6, **dadurch gekennzeichnet, dass** ein Drucksensor (100) vorgesehen ist, der derart angeordnet ist, dass er den Druck in dem arteriellen (60) und/oder in dem venösen Blutschlauch (70) misst und dass eine Steuereinrichtung vorgesehen ist, an die der Drucksensor (100) angeschlossen ist und die die Substituatpumpe (50) abschaltet, sobald der mittels des Drucksensors (100) erfaßte Druck einen Grenzwert übersteigt.

9. Verfahren nach einem der vorhergehenden Ansprüche, **dadurch gekennzeichnet, dass** von dem arteriellen (60) und/oder von dem venösen Blutschlauch (70) ein Leitungsabschnitt (110) abzweigt, dass dieser Leitungsabschnitt (110) mit dem arteriellen (60) oder venösen Blutschlauch (70) oder einem daran angeordneten Leitungsabschnitt verbunden wird und dass der arterielle (60) und/oder venöse Blutschlauch (70) stromabwärts der Einmündung des abzweigenden Leitungsabschnitts (110) abgesperrt wird.

10. Blutbehandlungsgerät mit extrakorporalem Kreislauf, geeignet zur Aufnahme eines Blutschlauchsatzes sowie eines Membranfilters mit einer ersten Kammer (20) und einer von dieser durch eine Membran getrennten zweiten Kammer (30), wobei die erste Kammer in Betrieb des Blutbehandlungsgerätes von Blut und die zweite Kammer (30) im Betrieb des Blutbehandlungsgerätes von Filtrat durchströmt wird, **dadurch gekennzeichnet, dass** das Blutbehandlungsgerät eine Substituatpumpe (50) und eine Steuereinheit aufweist, die die Substituatpumpe (50) zum Zwecke der Entleerung des Blutschlauchsatzes derart ansteuert, dass mittels der Substituatpumpe (50) Luft oder Gas in den Blutschlauchsatz gepumpt wird, so dass in dem Blutschlauchsatz ein Überdruck entsteht, durch den Flüssigkeit über die Membran des Membranfilters (10) von der ersten Kammer (20) in die zweite Kammer (30) des Membranfilters (10) verdrängt wird.

11. Blutbehandlungsgerät nach Anspruch 10, **dadurch gekennzeichnet, dass** die Substituatpumpe (50) derart ausgeführt ist, dass sie nach der Dekonnektion der Substituatleitung (40) von der Substituatquelle durch die Substituatleitung (40) hindurch Umgebungsluft ansaugt und in den Blutschlauchsatz fördert.

12. Vorrichtung nach Anspruch 10 oder 11, **dadurch gekennzeichnet, dass** eine Blutpumpe (80) vorgesehen ist und dass die Steuereinheit derart ausgeführt ist, dass sie die Blutpumpe (80) zum Entleeren des Blutschlauchsatzes derart ansteuert, dass die Blutpumpe (80) während des Entleerens des Blutschlauchsatzes permanent oder zeitweise in Betrieb ist.

13. Blutbehandlungsgerät nach einem der Ansprüche 10 bis 12, **dadurch gekennzeichnet, dass** die Steuereinheit derart ausgeführt ist, dass sie die Substituatpumpe (50) nach einer vorbestimmten Zeitspanne ab dem Einschaltzeitpunkt wieder abschaltet.

14. Blutbehandlungsgerät nach einem der Ansprüche 10 bis 13, **dadurch gekennzeichnet, dass** ein Drucksensor (100) vorgesehen ist, der an die Steuereinheit angeschlossen ist und der derart angeordnet ist, dass er den Druck in dem arteriellen (60) und/oder in dem venösen Blutschlauch (70) mißt, und dass die Steuereinheit derart ausgeführt ist, dass sie die Substituatpumpe (50) abschaltet, sobald der mittels des Drucksensors (100) erfaßte Druck einen Grenzwert übersteigt.

## Claims

1. A method for the emptying of a blood tubing set of a blood treatment device comprising an extracorporeal blood circuit including a membrane filter (10) having a first chamber (20), and a second chamber (30) separated therefrom by a membrane, said first chamber (20) being flowed through by blood in operation of the blood treatment device and said second chamber (30) being flowed through by filtrate in operation of the blood treatment device, and said blood tubing set including an arterial blood tube (60) and a venous blood tube (70) which are both in communication with the first chamber (20) of the membrane filter (10) and which conduct blood from the patient to the membrane filter (10) and from the membrane filter (10) to the patient in operation of the blood treatment device, and comprising a substituate line (40) which opens into the arterial blood tube (60) and/or into the venous blood tube (70), and comprising a substituate pump (50) which is in communication with the substituate line (40) such that it conveys substituate from a substituate source through the substituate line, **characterized in that,** for the purpose of the emptying of the blood tubing set, the arterial blood tube (60) and the venous blood tube (70) are connected to one another such that a circuit including the membrane filter (10), the arterial blood tube (60) and the venous blood tube (70) arises, that the substituate line (40) is disconnected from the substituate source and that air or gas is pumped into the blood tubing set by means of the substituate pump (50) so that an excess pressure arises in the blood tubing set by which liquid is displaced into the second chamber (30) of the membrane filter (10) via the membrane of the membrane filter (10).

2. A method in accordance with claim 1, **characterized in that** the substituate pump (50) is loaded with ambient air which is conveyed into the blood tubing set through the substituate pump (50).

3. A method in accordance with either of claims 1. or 2, **characterized in that** the substituate line (40) is made as a predilution line (42) and/or as a post-dilution line (44).

4. A method in accordance with one of the preceding claims, **characterized in that** the substituate line (40) branches into a predilution line (42) and into a post-dilution line (44); and **in that**, on the emptying of the blood tubing set, the predilution line (42) or the post-dilution line (44) is shut off so that the air compressed in the substituate pump (50) is only transported into the blood tubing set through the predilution line (42) or the post dilution line (44).

5. A method in accordance with one of the preceding claims, **characterized in that** a blood pump (80) is arranged which is in communication with one of the blood tubes, preferably with the arterial blood tube (60) such that, in the operation of the blood treatment device, blood is conveyed through the blood tubing set; and **in that** the blood pump (80) is in operation permanently or at times during the emptying of the blood tubing set.

6. A method in accordance with one of the preceding claims, **characterized in that** the air conveyed by means of the substituate pump (50) is conveyed via the predilution line (42) or the post-dilution line (44) into the arterial blood tube (60) or the venous blood tube (70) and is split in the latter such that the air or the liquid displaced by the air enters into the first chamber (20) of the membrane filter (10) both via the arterial blood tube (60) and via the venous blood tube (70).

7. A method in accordance with one of the preceding claims, **characterized in that** the substitute pump (50) is switched off again after a predetermined time period from the switch-on time.

8. A method in accordance with one of the claims 1 to 6, **characterized in that** a pressure sensor (100) is provided which is arranged such that it measures the pressure in the arterial blood tube (60) and/or in the venous blood tube (70); and **in that** a control device is provided to which the pressure sensor (100) is connected and which switches the substituate pump (50) off as soon as the pressure detected by means of the pressure sensor (100) exceeds a threshold.

9. A method in accordance with one of the preceding claims, **characterized in that** a line section (110) branches off from the arterial blood tube (60) and/or from the venous blood tube (70); **in that** this line section (110) is connected to the arterial blood tube (60) or to the venous blood tube (70) or to a line section arranged thereon; and **in that** the arterial blood tube (60) and/or the venous blood tube (70) is shut off downstream of the junction of the branching off line section (110).

10. A blood treatment device comprising an extracorporeal circuit suitable for the reception of a blood tubing set as well as of a membrane filter, having a first chamber (20) and a second chamber (30) separated therefrom by a membrane, said first chamber being flowed through by blood in operation of the blood treatment device and said second chamber (30) being flowed through by filtrate in operation of the blood treatment device, **characterized in that** the blood treatment device has a substituate pump (50) and a control unit which controls the substituate pump (50) for the purpose of emptying the blood tubing set such that air or gas is pumped into the blood tubing set by means of the substituate pump (50) such that excess pressure arises in the blood tubing set by which liquid is displaced from the first chamber (20) into the second chamber (30) of the membrane filter (10) via the membrane of the membrane filter (10).

11. A blood treatment device in accordance with claim 10, **characterized in that** the substituate pump (50) is made such that it sucks in ambient air through the substituate line (40) and conveys it into the blood tubing set after the disconnection of the substituate line (40) from the substituate source.

12. An apparatus in accordance with either of claims 10 or 11, **characterized in that** a blood pump (80) is provided; and **in that** the control unit is made such that it controls the blood pump (80) for the emptying of the blood tubing set such that the blood pump (80) is in operation permanently or at times during the emptying of the blood tubing set.

13. A blood treatment device in accordance with one of the claims 10 to 12, **characterized in that** the control unit is made such that it switches the substituate pump (50) off again after a predetermined time period from the switch-on time.

14. A blood treatment device in accordance with one of the claims 10 to 13, **characterized in that** a pressure sensor (100) is provided which is connected to the control unit and which is arranged such that it measures the pressure in the arterial blood tube (60) and/or in the venous blood tube (70); and **in that** the control unit is made such that it switches off the substituate pump (50) as soon as the pressure detected by means of the pressure sensor (100) exceeds a threshold.

## Revendications

1. Procédé de purge d'un jeu de lignes à sang d'un appareil de traitement sanguin avec une circulation extracorporelle du sang avec un filtre à membrane (10) qui présente une première chambre (20) et une deuxième chambre (30) séparée de celle-ci par une membrane, où la première chambre (20), lors du fonctionnement de l'appareil de traitement sanguin, est traversée par le sang, 1 et où la deuxième chambre (30), lors du fonctionnement de l'appareil de traitement sanguin, est traversée par le filtrat, et où le jeu de lignes à sang comprend un tuyau de sang artériel (60) et un tuyau de sang veineux (70), qui sont reliés tous les deux à la première chambre (20) du filtre à membrane (10) et qui, lors du fonctionnement de l'appareil de traitement sanguin, guident le sang du patient au filtre à membrane (10) et du filtre à membrane (10) au patient, et avec un conduit de substituat (40) de telle sorte qu'il guide le substituat d'une source de substituat à travers le conduit de substituat (40), **caractérisé en ce que** dans le but de la purge du jeu de lignes à sang, le tuyau de sang artériel (60) et le tuyau de sang veineux (70) sont reliés de manière à former une circulation comprenant le filtre à membrane (10), le tuyau de sang artériel (60) et le tuyau de sang veineux (70), que le conduit de substituat (40) est déconnecté de la source de substituat, et qu'au moyen de la pompe de substituat (50), de l'air ou du gaz est pompé dans le jeu de lignes à sang de façon à produire dans le jeu de lignes à sang une surpression par laquelle du liquide est refoulé par la membrane du filtre à membrane (10) dans la deuxième chambre (30) du filtre à membrane (10).

2. Procédé selon la revendication 1, **caractérisé en ce que** la pompe de substituat (50) est chargée en air ambiant qui est convoyé par la pompe de substituat (50) dans le jeu de lignes à sang.

3. Procédé selon la revendication 1 ou 2, **caractérisé en ce que** le conduit de substituat (40) est réalisé comme conduit de prédilution (42) et/ou comme conduit de postdilution (44).

4. Procédé selon l'une des revendications précédentes, **caractérisé en ce que** le conduit de substituat (40) se ramifie en un conduit de prédilution (42) et un conduit de postdilution (40), et **en ce que** lors de la purge du jeu de lignes à sang, le conduit de prédilution (42) ou le conduit de postdilution (44) est fermé de sorte que l'air comprimé dans la pompe de substituat (50) est convoyé seulement à travers le conduit de prédilution (42) ou le conduit de postdilution (44) dans le jeu de lignes à sang.

5. Procédé selon l'une des revendications précédentes, **caractérisé en ce qu'**une pompe de sang (80) est disposée qui est reliée à un des tuyaux de sang, de préférence au tuyau de sang artériel (60) de façon qu'en cours de fonctionnement de l'appareil de traitement sanguin, le sang soit convoyé à travers le jeu de lignes à sang et **en ce que** la pompe de sang (80), pendant la purge du jeu de lignes à sang, fonctionne en permanence ou temporairement.

6. Procédé selon l'une des revendications précédentes, **caractérisé en ce que** l'air convoyé par la pompe de substituat (50) est convoyé par le conduit de prédilution (42) ou le conduit de postdilution (44) dans le tuyau de sang artériel (60) ou veineux (70) et est réparti dans celui-ci de façon que l'air respectivement le liquide refoulé par l'air entre à la fois par le tuyau de sang artériel (60) et aussi par le tuyau de sang veineux (70) dans la première chambre (20) du filtre à membrane (10).

7. Procédé selon l'une des revendications précédentes, **caractérisé en ce que** la pompe de substituat (50), après une durée de temps prédéterminée à partir de l'instant de mise en service, est à nouveau mise hors service.

8. Procédé selon l'une des revendications 1 à 6, **caractérisé en ce qu'**un capteur de pression (100) est prévu qui est disposé de telle sorte qu'il mesure la pression dans le tuyau de sang artériel (60) et/ou dans le tuyau de sang veineux (70), et **en ce qu'**une installation de commande est prévue à laquelle le capteur de pression (100) est raccordé et qui met hors service la pompe de substituat (50) dès que la pression détectée par le capteur de pression (100) dépasse une valeur limite.

9. Procédé selon l'une des revendications précédentes, **caractérisé par** la séparation du tuyau de sang artériel (60) et/ou du tuyau de sang veineux (70) d'une section de conduit (110), en ce que cette section de conduit (110) est reliée au tuyau de sang artériel (60) ou veineux (70) ou une section de conduit disposée à celui-ci, et en ce que le tuyau de sang artériel (60) et/ou veineux (70) est fermé en aval de l'embouchure de la section de conduit de dérivation (110).

10. Appareil de traitement sanguin avec une circulation extracorporelle, qui convient à la réception d'un jeu de lignes à sang ainsi que d'un filtre à membrane avec une première chambre (20) et une deuxième chambre (30) séparée de celle-ci par une membrane, où la première chambre, lors du fonctionnement de l'appareil de traitement sanguin, est traversée par le sang et la deuxième chambre (30), lors du fonctionnement de l'appareil de traitement sanguin, par du filtrat, **caractérisé en ce que** l'appareil de traitement sanguin comprend une pompe de substituat (50) et une unité de commande qui commande la pompe de substituat (50) dans le but de la purge du jeu de lignes à sang de telle sorte qu'au moyen de la pompe de substituat (50), de l'air ou du gaz est pompé dans le jeu de lignes à sang de sorte que dans le jeu de lignes à sang, une surpression est produite par laquelle du liquide est refoulé par la membrane du filtre à membrane (10) de la première chambre (20) dans la deuxième chambre (30) du filtre à membrane (10).

11. Appareil de traitement sanguin selon la revendication 10, **caractérisé en ce que** la pompe de substituat (50) est réalisée de telle sorte qu'après la déconnection du conduit de substituat (40) de la source de substituat, elle aspire de l'air ambiant à travers le conduit de substituat (40) et le convoie dans le jeu de lignes à sang.

12. Dispositif selon la revendication 10 ou 11, **caractérisé en ce qu'**une pompe de sang (80) est prévue, et **en ce que** l'unité de commande est réalisée de telle sorte qu'elle commande la pompe de sang (80) pour la purge du jeu de lignes à sang de telle sorte que la pompe de sang (80), pendant la purge du jeu de lignes à sang, fonctionne en permanence ou temporairement.

13. Appareil de traitement sanguin selon l'une des revendications 10 à 12, **caractérisé en ce que** l'unité de commande est réalisée de façon à mettre hors service la pompe de substituat (50) après un laps de temps prédéterminé à partir de l'instant de mise en service.

14. Appareil de traitement sanguin selon l'une des revendications 10 à 13, **caractérisé en ce qu'**un capteur de pression (100) est prévu qui est raccordé à l'unité de commande et qui est disposé de façon à mesure la pression dans le tuyau de sang artériel (60) et/ou dans le tuyau de sang veineux (70), et **en ce que** l'unité de commande est réalisée de telle sorte qu'elle met hors service la pompe de substituat (50) dès que la pression détectée par le capteur de pression (100) dépasse une valeur limite.
